# EUROPEAN PATENT SPECIFICATION

(11) **EP 2 981 604 B1**
(45) Date of publication and mention of the grant of the patent: **18.04.2018**
(21) Application number: 14799564.1
(22) Date of filing: 01.10.2014
(51) Int. Cl.: C12M 1/00, B01D 53/62, C12M 1/12, B01D 53/84

(54) **PHOTOBIOREACTOR FOR CO2 BIOSEQUESTRATION WITH IMMOBILISED BIOMASS OF ALGAE OR CYANOBACTERIA**
FOTOBIOREAKTOR FÜR CO2-BIOSEQUESTRIERUNG MIT EINER IMMOBILISIERTEN BIOMASSE AUS ALGEN UND CYANOBAKTERIEN
PHOTOBIORÉACTEUR SERVANT À LA BIOSÉQUESTRATION DU CO2 COMPORTANT UNE BIOMASSE D'ALGUES OU DE CYANOBACTÉRIES

(30) Priority: 28.03.2014 PL 40769414
(43) Date of publication of application: 10.02.2016
(73) Proprietor: Uniwersytet Warminsko - Mazurski w Olsztynie, 10-179 Olsztyn - Kortowo (PL)
(72) Inventor: KRZEMIENIEWSKI, Miroslaw, 10-685 Olsztyn (PL); DEBOWSKI, Marcin, 10-686 Olsztyn (PL); ZIELINSKI, Marcin, 10-699 Olsztyn (PL)
(74) Representative: Wazynska, Miroslawa
(86) International application number: PCT/PL2014/000110
(87) International publication number: WO 2015/147661

(56) References cited:
- DE-A1-102007 035 707
- DE-A1-102010 025 366
- JP-A- H0 957 058

## Description

The object of the invention is a photobioreactor for CO₂ biosequestration with immobilised biomass of algae or cyanobacteria.

Phototrophic algae, using energy from sunlight or artificial light, via photosynthesis, convert atmosphere CO₂ into organic compounds which constitute their building material. Sufficient access to light and CO₂ is necessary for carrying out the photosynthesis and for the production of algae biomass. Further, pH value and temperature of the culture, as well as type of reactor, open or closed, are important parameters.

Due to the excessive global CO₂ emissions into the atmosphere and the need to conduct intensive and innovative sequestration of CO₂ coming from different sources, for the protection of human life environment and for the protection against the greenhouse effect, an effective method for binding CO₂ into biomass is constantly being searched for, to preserve the ecological balance in the nature.

One direction of this search is aimed at photobioreactors, and in particular closed photobioreactors, in which the culture of algae or cyanobacteria, conducted under control, is able to bind CO₂ into its own biomass. Various structures of closed photobioreactors allow monitoring and controlling of light intensity and exposure time, pH value and temperature of the culture, and purity and lifetime of the culture of a selected strain of algae or cyanobacteria.

Photobioreactors are already known in the art.

From U.S. patent application US 20080286857, a multi-functional airlift bioreactor, comprising cells entrapped in polymeric spheres for absorbing gases (volatile organic compounds) or odours, wherein the bioreactor is equipped with a sprinkling device, is known.

From another U.S. patent application US 5073491A, a method for culturing cells in an airlift bioreactor, the cells cultured herein being immobilised in alginate-based spheres, is known.

Chinese application CN 101240270 discloses alginate-based capsules with immobilised cells and a method for their preparation.

Japanese application JP 3112481 discloses a device for culturing algae, comprising a culture chamber, a rotating basket and a bundle of optical fibres. Gaseous carbon dioxide and sunlight are delivered evenly into the culture chamber by means of rotation of the rotating basket.

A method for immobilising microbial cells in the capsules with sodium alginate is known, for example, from PL 213167B1.

DE102007035707 A1 relates to a possibility of using immobilised algae biomass in water purification processes, mainly for aquaculture. It is proposed to place selected microalgae in a polymeric envelope and use of this type small structures for biofiltration of water coming mainly from fish farming systems. The function of immobilised algae biomass is to remove contaminants from water, the algae biomass being immersed in the liquid. A system for water biofiltration is proposed herein. DE102007035707 discloses a method of oxygenation and absorption of water contaminants, which comprises culturing microalgae, immobilising them in stable alginate-based envelopes having a diameter of 0.1 to 5 mm. Alginate-based spheres comprising microalgae and physiological solution are additionally placed in a transparent porous membrane having a pore size in the range of 0.5-50 µm. The membrane with alginate-based spheres is placed in the photobioreactor which is fed with contaminated water. The photobioreactor is equipped with two LED light sources and light is supplied into entire volume of the liquid in the fotobioreactor. Information contained in this document could not constitute a basis to develop technologies for removing carbon dioxide from waste gases.

US 5073491 describes a method for cell production in an alginate bed in an airlift-type reactor. The only common feature of both solutions is immobilisation of cells in the alginate gel. However, the use of sodium alginate as a carrier for different types of biological forms has been commonly known for a long time.

Known technology solutions in the systems for biosequestration of carbon dioxide based on the use of algae biomass, which include open bioreactors or closed photobioreactors, where the algae biomass is placed in aqueous solutions, do not allow achieving satisfactory results of CO₂ removal, from the point of view of installations operated on a technical scale. This is due, among others, to the phenomenon of carbon dioxide solubility in water, which may cause a decrease in pH value, i.e. acidification of the culture medium to a level below that required by algae or cyanobacteria for their growth and life. The functioning efficiency of classical systems based on the use of biomass of algae or cyanobacteria is limited also by the inhibition of light transmission by aqueous culture medium, which reduces growth rate of the biomass and efficiency of CO₂ removal. For these reasons, known algae systems may be introduced with small amounts of gases containing carbon dioxide, or they require very large surfaces (such as open ponds) or cubatures (closed photobioreactors). Efficiency of CO₂ removal is further limited by the formation and accumulation in the environment of gaseous metabolic products of these organisms in the form of oxygen and carbon dioxide introduced into the aqueous environment.

The aim of the invention is to create a new structure of a device for removing CO₂ from the flue gases and the waste gases coming from different industry sectors, e.g. from the food industry from yeast production process, biogas production systems, liquid and gaseous fuels combustion systems, hydrocarbon processing technology. The solution according to the invention allows effective removal of carbon dioxide from gases at a high CO₂ concentration. At the same time, the device is characterised by a significantly lower cubature in relation to the currently used solutions which use CO₂ biosequestration processes.

The invention, thanks to its structure, assumes achieving a very high concentration of biomass of algae or cyanobacteria through its immobilisation in capsules. This eliminates the phenomenon of lowering the pH value, i.e. acidification of the environment in which algae or cyanobacteria grow, resulting from the introduction of waste gases at a high CO₂ concentration.

The system for introducing light into algae or cyanobacteria capsules is characterised by a higher degree of its use, due to lower losses resulting from the phenomenon of absorption of light energy by water. As a result of the cyclic dosing of gases containing carbon dioxide, gaseous metabolic products of algae or cyanobacteria are displaced and removed outside the technological system, thereby eliminating the negative impact of this factor on CO₂ binding processes. The periodic introduction of the culture medium with a high concentration of nutrients contributes to its better use, and the flushing process used allows systematic removal of excessive biomass outside the device.

Biomass obtained in this technological process, can be used for various purposes, mainly as animal feed (food for fish, culture medium for zooplankton), fertilizers and in power industry (substrate for biogas systems, source of bio-oil).

The subject of the invention is a photobioreactor for CO₂ biosequestration with immobilised biomass of algae or cyanobacteria, having a closed structure divided into segments and capsules of biomass of algae or cyanobacteria, wherein each of the capsules of biomass of algae or cyanobacteria (3) has a diameter between 5 mm and 40 mm and is individually supplied with light from a light source (6) by light tubes (5). The capsules of biomass of algae or cyanobacteria (3) in the photobioreactor segment lie freely on a perforated grid (2). The light source (6) is transferred via a separate single light tube (5) to the inside of each capsule of biomass of algae or cyanobacteria (3). Preferably the capsules of biomass of algae or cyanobacteria (3) are surrounded by a gaseous atmosphere and they are wetted with a culture medium. Preferably these capsules (3) are periodically wetted and flushed with the culture medium.

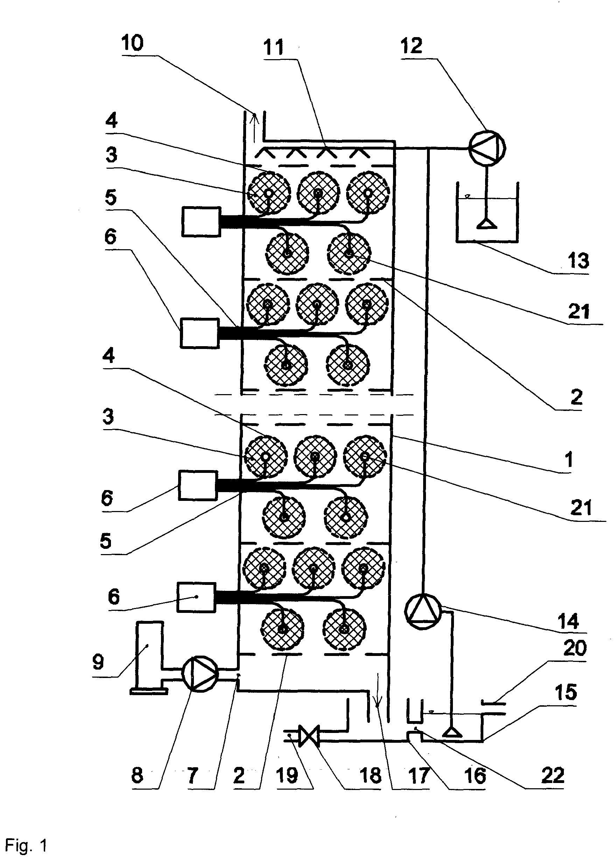

The object according to the invention is shown in the drawing in which
Fig. 1 is a general scheme of the photobioreactor,

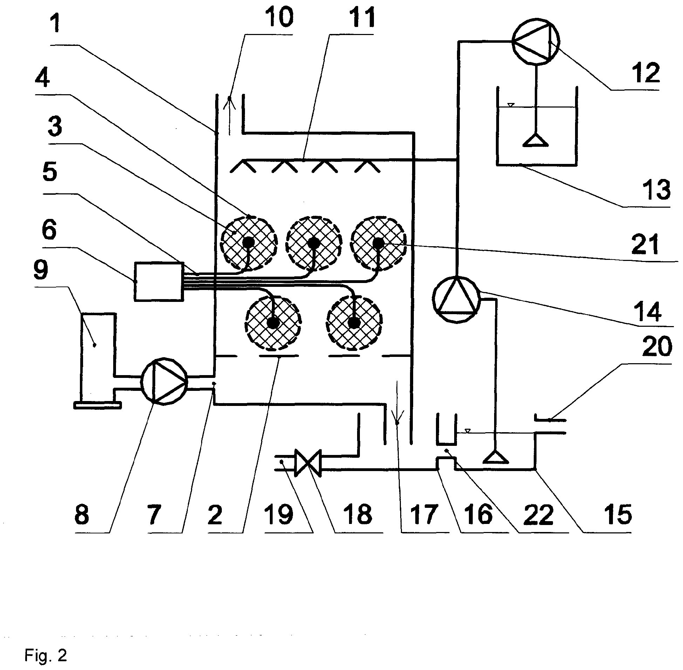

Fig. 2 is a scheme of the photobioreactor constructed of one segment,

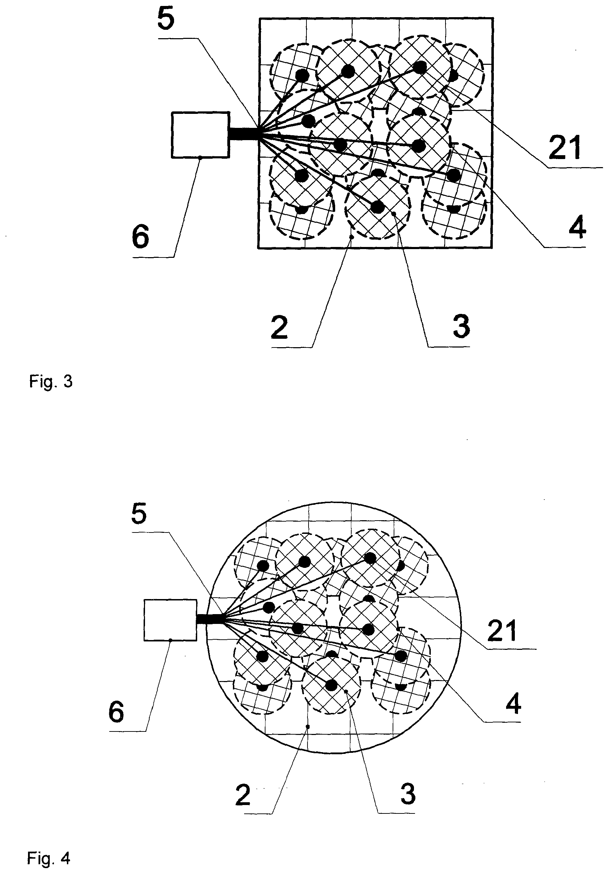

Fig. 3 is a cross-sectional scheme of the polyhedral photobioreactor, and
Fig. 4 is a cross-sectional scheme of the circular photobioreactor.

The capsule is formed from biomass of algae or cyanobacteria by two different methods. The first method involves the use of a perforated gel envelope. The biomass of algae or cyanobacteria, obtained from the culture, is subjected to a sieve selection, using a microsieve, so that the size of algae or cyanobacteria correspond to the size of the openings in the gel envelope. Selected biomass is concentrated and dehydrated to a level of 8%-15% of dry matter, which allows obtaining formable plastic mass. Then, a shape close to a sphere having a diameter in the range of 4.5 mm to about 40 mm is formed. Into the so formed biomass of algae or cyanobacteria a light tube of a diameter of 0.7 mm to 3.0 mm is introduced. If the diameter of the capsule does not exceed 15 mm, then a light tube without light beam scattering material on its end may be used, and if the diameter is larger, then the end of the light tube is equipped with a light beam scattering material, made of acrylic mass or of glass with a diameter at least 2-fold larger than the diameter of the light tube. Then, the formed biomass of algae or cyanobacteria is covered with a gelling agent which is, for example, sodium alginate. In order to made the envelope porous, with a pore size of 5 µm to 100 µm, the gelling agent is added with a pore-forming material which, when dissolved and flushed, will create pores of an expected diameter. For algae or cyanobacteria preferring sweetness, crystallised glucose with a crystal size corresponding to the sizes of the expected pores is proposed. For algae or cyanobacteria preferring saltiness, this may be glucose or crystallised sodium chloride. The amount of the pore-forming material in relation to the gelling agent does not exceed 40% of volume due to the maintenance of mechanical strength of the envelope formed. The pore-forming material dissolves after 1 to 5 hours since its addition to the gelling agent.

The second method of forming the capsule involves condensation and dehydration of biomass of algae or cyanobacteria so as to obtain 5.0% to 10% of dry matter. Then, the biomass of algae or cyanobacteria is introduced to the enveloped formed from a plastic material which is porous with openings of 5 µm to 100 µm. It should be noted that algae or cyanobacteria, which are intended to be introduced, before condensation and dehydration, to the envelope, are subjected to a sieve selection in respect of their size, and their size must be equal to or slightly larger than the pore size in the capsule envelope. Having filled the envelope with biomass of algae or cyanobacteria, a light tube of a diameter of 0.7 mm to 3.0 mm is introduced. Thus, in the case of a plastic envelope, if the capsules have diameters larger than 15 mm, the tube ends with a scattering acrylic or glass material. The diameter of the scattering material is 2-fold larger than the diameter of the light tube. The opening, through which the biomass and the light tube with the scattering material were introduced, is closed with a removable sealing material so as to allow easy removal and so as to allow performing again the procedure of filling the capsule in the case of an excessive amount of biomass of algae or cyanobacteria flowing out of it.

The photobioreactor according to the invention has the following structure:
The housing of the photobioreactor device (1) has the shape of a tower tank, divided by partitions into segments of a number of one to several hundred segments. The dividing partitions are perforated grids (2) on which the biomass of algae or cyanobacteria is placed in the form of capsules (3) which have a diameter of 5 mm to 40 mm in their cross section. For the selection of capsule diameter, type of gases, type of algae or cyanobacteria, number of capsule layers, light intensity and the like are decisive.

The outer envelope (4) of the capsule (3) is made of a porous material having a pore size of 5 µm to 100 µm, which allows the excessive biomass of algae or cyanobacteria to flow out of the capsule (3). The outer envelope (4) may be created on the biomass of algae or cyanobacteria previously formed in the form of a capsule, for example by coating it with gel mass or by introducing the biomass of algae or cyanobacteria to the prepared outer envelope (4), for example, in the form of a perforated plastic material. Into the inside of the biomass of algae or cyanobacteria in capsule (3), a light tube (5) is brought which is ended with a tip (21) of a material scattering light throughout the capsule (3) in the direction of its outer envelope (4). The second end of the light tube (5) is connected to the light source (6).

Below the lowermost perforated grid (2), in the housing of the photobioreactor device (1), there is a conduit inlet (7) for a CO₂ containing gas, which is connected to a pump (8) conveying the gas with CO₂ from a retention tank (9) that contains CO₂.
In the overhead protection in the housing of the device (1), an outlet duct (10) for gases is mounted.

Sprinkler nozzles (11) are located above the biomass of algae or cyanobacteria in capsules (3) and have a connection to a culture medium dosing pump (12) and to a culture medium tank (13), as well as to a flushing pump (14) and to a retention tank (15) with clarified water.

The retention tank (15) is fluidly connected by a conduit (22) to a separation tank (16) for the excessive biomass of algae or cyanobacteria.

An outlet duct (17) discharges the excess of the biomass of algae or cyanobacteria from the bottom of the photobioreactor device (1) to the separation tank (16) for the excessive biomass of algae or cyanobacteria and this excess of the biomass is discharged further, outside the photobioreactor, by a drain conduit (19) with a valve (18).

The formed biomass of algae or cyanobacteria (3) has the shape of capsules of a diameter of 5 mm to 40 mm and is covered with the outer envelope (4) having a perforation of a diameter of 5 µm to 100 µm. The outer envelope (4) is in the form of a layer of gel substance or a layer formed from a perforated plastic coating.

The light is supplied with a separate light tube (5) into the biomass of algae or cyanobacteria to each capsule (3). Into the biomass of algae or cyanobacteria in capsule (3), gases containing CO₂ and liquid culture medium which is periodically fed from the top, from the sprinkler nozzles (11), are periodically introduced.

The generated excessive algal biomass is periodically flushed from the top, directed to the separation tank (16) and discharged outside the photobioreactor, by the drain conduit (19).

The light from the light source (6) is continuously supplied to the formed biomass of algae or cyanobacteria in capsules (3) by the light tubes (5). The light source (6) may be sunlight or a light generator of different wavelength of light from 300 nm to 800 nm.

A portion of gas containing CO₂, accumulated in the tank (9) and conveyed by the pump (8) is periodically introduced into the housing of the photobioreactor (1) tank. When conveying the gas that contains CO₂, it penetrates the algal biomass in capsules (3) through the outer envelope (4), displacing, from the algal biomass, gaseous metabolic products which are discharged by the outlet duct (10) for gases.

When the pump (8) completes its operation, the pump (12) starts operating, through which the liquid culture medium is conveyed from the culture medium tank (13) to the sprinkler nozzles (11). The culture medium flows over outer surfaces of the envelopes (4) and penetrates into the algal mass in capsules (3), contributing, combined with the supplied light energy and CO₂, to the increase of the algal biomass. The algal biomass escapes from the capsules (3) through the perforated outer envelope (4) and is periodically flushed with the liquid, when pumping the liquid with the flushing pump (14) from the retention tank (15). The liquid was obtained after the previous separation of the excessive algal biomass in the separation tank (16) into which it flows from the entire volume of the housing of the photobioreactor device (1), together with the flushing liquid, by the outlet duct (17). After periodical opening of the valve (18), the condensed excessive algal biomass is removed outside the photobioreactor by the conduit (19), and the mixture of unused liquid medium and liquid metabolic products flows out partially by an outflow duct (20) outside the photobioreactor and partially returns into circulation, pumped by the flushing pump (14).

### Example 1

The photobioreactor device was made on a laboratory scale. An experiment was conducted to determine its effectiveness. The housing of the photobioreactor device 1 was a pipe of a transparent plastic. The outer surface of the photobioreactor was covered with a dark film impermeable to either sunlight or artificial light, prevailing in the laboratory. There was a possibility to remove the film to observe the condition of the capsules and the situation inside the housing of the photobioreactor device 1. Internal dimensions of the housing of the photobioreactor device 1 were respectively: diameter 30 mm, height 1000 mm. A support grid 2 with a mesh size of 5 mm was positioned at a height of 50 mm above the lower base of the housing of the photobioreactor device 1. The lower base of the housing was immersed 15 mm below the water surface in the tank 16, with a capacity of 1 dm³, from which excess of water flowed out through the conduit 22 to a second collecting vessel, with a capacity of 1 dm³, i.e. to the retention tank 15. At the top of the housing of the photobioreactor device 1, the gas outlet duct 10 in the form of a conduit of a diameter of 5 mm was located, from which escaping gases were collected in a tedlar bag, from where these gases were collected for analysis. The gas analysis was conducted by means of a gas chromatograph Agilent 7980A. The second conduit in the upper part of the housing of the device 1, together with a perforated plastic mesh, mesh size 2 mm, fulfil the function of a sprinkler 11 connected to the conduit which doses, together, the culture medium and flushing water from the second collecting vessel.
The layer of the algae biomass capsules 3 placed inside the housing 1 had a thickness of 800 mm. The capsules 3 had a diameter of 8 mm. To prepare the capsules, *Chlorella protothecoides* algae biomass, strain SLYCP01, from own culture in photobioreactors with a volume of 3 m³, illuminated by both sunlight and artificial light, was used. Before placing the capsules in the photobioreactor, the algae biomass was concentrated on mesh filters, mesh size 10 µm, and then dehydrated on a centrifuge. After dehydration, the algae were in the form of plastic mass. It was formed into capsules 3, coated with a mass of sodium alginate. After formation, the capsules 3 had a diameter of 8 mm, and into each capsule the light tube 5 was introduced, which in this case, due to the small diameter of the capsules, was not ended with light scattering mass. The other ends of the light tubes 5 were placed in a light source 6, here in an artificial light source, a lamp emitting white light. The algae capsules 3 were arranged in a loose mound. In the lower part of the housing, below the perforated grid 2 supporting the capsules 3, there was a CO₂ conduit inlet 7 which was controlled by a pump 8. The gas was constituted by atmospheric air, enriched with technically pure CO₂ so that the concentration of CO₂ was 25% v/v. The culture medium for algae was prepared in a separate tank 13 and was dosed by the pump 12. Dosing of the culture medium and the gas with CO₂ took place alternately, every 10 minutes for 1 minute. The capsules were flushed once a day.

The effectiveness of CO₂ retention in the device was determined by measuring CO₂ concentration in the input gas and the amount of CO₂ in the output gas. The efficiency of CO₂ removal was about 80%.

In a typical photobioreactor, it is not possible to use such a high CO₂ concentration because the phenomenon of aqueous environment acidification occurs quickly. With a high concentration of biomass, in an exemplary device, acidification, or decrease in pH value, does not occur because CO₂ is immediately used by the algae biomass.

Volumetric efficiency of CO₂ absorption obtained in an exemplary reactor is 15 fold larger than in other photobioreactors.

### Example 2

The housing of the device 1 consists of four side walls, a flat overhead protection and a flat floor sloped at 2% towards the outflow opening. The height of the device housing, according to the internal dimension from the floor to the overhead protection, is 5.1 m, and in cross section the internal dimensions are respectively: length 2.5 m, width 1.0 m. Three vertical walls are transparent, of 3 mm glass with vacuum-insulation. The fourth wall is located on the north side. This is a steel wall, made of acid resistant steel and with thermal insulation of foamed polystyrene with a thickness of 80 mm. Vertical walls of the housing are divided horizontally, evenly into 20 segments each 0.2 m high, and into two end segments with a height of 0.3 m each - the upper one, ended with the overhead protection and the lower one, ended with the flat floor. Each of the 20 segments is an independent structural element consisting of a side wall, with thermal insulation, permanently connected to the perforated grid 2 of plastic with a thickness of 8.0 mm, which is arranged on a guide that allows its sliding, and removing the grid outside the housing of the device to periodically change its place according to the rule that the highest grid is successively moved to the place of the lowest one. Perforation produced during the construction of the grid occupies 60% of the grid surface and the openings have a diameter of 10 mm. The edges of the perforated grid have a height of 50 mm, which protects the capsules from falling out when moving the grid. The capsules 3, in the number of 32,000 pcs for each segment, are arranged in the form of a loose mound (in the whole device, there are about 640 000 capsules), which facilitates flushing of excessive biomass of algae or cyanobacteria. The mound takes the form of an envelope with a height of 150 mm, and from the edge of the grid the mound increases at a ratio of 2/1. The capsules 3 with a gel envelope 4 of a diameter of 25 mm comprise, in their interior, acrylic light-scattering mass of a diameter of 5 mm which is connected to the tip of the light tube 5 of a diameter of 1.5 mm. The light tubes 5 from each capsule are led through the side wall with thermal insulation to the light source 6, fixed to it, with a wave length of 640 nm, with a power of 200 W. In the upper segment with a height of 0.3 m, on the upper housing of the device, that is in its overhead protection of the bioreactor, 24 sprinkler nozzles 11 are permanently mounted in the form of full cone nozzles. The nozzles 11 ensure distribution of water or water with culture medium for flushing over the whole surface. Each nozzle is connected to a pressure conduit of a diameter of 25 mm, connected to a main conduit of a diameter of 50 mm. All the conduits are fixed to the overhead protection of the housing 1. Then, the main conduit of 50 mm, extending outside the housing of the device, is insulated with a thermal insulation and is connected by a tee to the submersible conveying pump 12, with a power of 0.2 kW with a check valve, the efficiency of which is Q= 0.001 m³/min, and the lifting height is H = 10 m H₂O. The second end of the tee is connected to the pressure conduit of a diameter of 50 mm and to a 0.5 kW submersible pump by a check valve 14, the efficiency of which is Q= 0.01 m³/min, and the lifting height is H = 30 m H₂O. The pump 12 is located in the tank 13 with culture medium with a volume of 0.2 m³ and is placed at the height of the last, highest segment of the housing of the device 1. Both pumps 12 and 14 have controllers to determine operating time for each pump. For the pump 12, culture medium pumping time of 0.5 minute and pause time of 10 minutes are adopted. The other pump 14 pumps water from the retention tank 15 every 6 hours for 8 minutes and this may take place only with the interruption of pumping of water with culture medium. The volume of the retention tank is 0.5 m³ and it is a flow tank, on one side it is connected to the separation tank 16, and on the other side it is ended with the outflow duct 20 of a diameter of 200 mm, in order to discharge excess of water which, after purification process in separate devices, returns to the tank 13 and is used to prepare a solution with mineral culture media. Concentration of the culture medium is 50 fold larger than the culture medium content during culture of algae or cyanobacteria in conventional opened culture tanks. Gases in an amount of about 1000 m³ per day after burning biogas are cooled to a temperature of 30°C and are collected in the tank 9 of a volume of 30 m³ and periodically, every 9 minutes, for 1 minute, are introduced to the lower segment of the housing of the device 1. Flue gases from the tank 9 flow through a duct of a diameter of 300 mm to the gas conveying pump 8, in the form of a blower, at a capacity rate of Q_{g}= 7.0 m³/min. The blower outlet is connected to the CO₂ conduit inlet designed as a diffuser, which when combined with the housing of the device 1, has dimensions of rectangle 100 mm x 1500 mm and is mounted 100 mm below the lowest perforated grid 2.

Further, the gas outlet duct 10 of the device is located in the overhead protection of the device in the upper segment, at the steel housing, and it is a pipe 0.3 m high and of a diameter of 0.5 m, secured from the top by a steel mesh, mesh size 5 mm x 5 mm.

The floor of the housing of the device 1 is made of acid resistant steel and is inclined by 2% towards the outlet duct 17, through which cultured biomass of algae or cyanobacteria and aqueous liquid, containing both unused culture medium substances and metabolic products produced during the process of photosynthesis, flow out.

The outlet duct 17 of a diameter of 200 mm is immersed at its other end in the water in the separation tank 16 for excess of biomass of algae or cyanobacteria of a volume 0.5 m³, forming a siphon closure which does not allow discharge, through this channel, of gases introduced into the device. The tank 16 is integrated with the valve 18, and on its other end, the drain conduit 19 of a diameter of 200 mm is mounted, through which condensed biomass of algae or cyanobacteria is discharged, and in an embodiment, it is directed to an about 30 kW agricultural biogas plant.

The daily amount of biogas produced in the fermentation process of organic substrates in this type of installation is about 100 m³ a day.

Qualitative composition of biogas is as follows:
methane - 66% v/v,
CO₂ - 33% v/v,
other gases about 1% v/v.

The biogas is burned in a gas boiler, and the amount of flue gases produced is at a level of about 1000 m³ a day with 14% v/v of CO₂ content. The daily amount of carbon dioxide produced is about 30 kg of CO₂ a day.

Efficiency of carbon dioxide biosequestration in the device according to the invention is 80% and results in CO₂ removal from flue gases at a level of 24 kg of CO₂ a day. The volume of the device filled with capsules is about 6m³ at a concentration of microalgae biomass in capsules at a level of 22 kg d.m./m³.

The total quantity of dry matter of microalgae in the device is about 130 kg. Efficiency of algae biomass growth in the device is in the range of 8.2 - 8.6 kg d.m./m³ a day.

As a result of CO₂ carbon dioxide binding and the use of nutrients delivered to the technological system, overall growth of algae biomass is in the range of about 50 kg d.m. a day.

The obtained biomass of microalgae will constitute a substrate for the biogas plant.

From 50 kg of microalgae dry matter, about 25 m³ of biogas with about 70% of methane content can be obtained, which satisfies internal purposes of the biogas plant for organic substrate in about 25% and provides a potential power at a level of 7.2 kW.

In comparison, in typical open photobioreactors, concentration of microalgae biomass is at a level of about 3 kg d.m./m³, which is a value over 7 times lower with respect to the presented device. Further, the rate of growth of algae biomass is at a level of about 0,25 kg d.m./m³ a day. This means that to obtain 50 kg d.m. of microalgae a day, which is a prerequisite for removing 24 kg of CO₂ a day, in the device with a typical depth of 0.3 m, a surface of about 600 m² is required.

### List of numeral references

(1) housing of the photobioreactor device
(2) perforated grids
(3) capsule of biomass of algae or cyanobacteria
(4) outer envelope of the capsule
(5) light tube
(6) light source
(7) CO₂ conduit inlet
(8) gas conveying pump
(9) CO₂ tank
(10) gas outlet duct
(11) sprinkler nozzles
(12) culture medium dosing pump
(13) tank with culture medium
(14) flushing pump
(15) retention tank
(16) separation tank for excess of biomass of algae or cyanobacteria
(17) outlet duct
(18) valve
(19) drain conduit
(20) outflow duct
(21) tip of the light tube inside the capsule
(22) conduit

## Claims

1. A photobioreactor for CO₂ biosequestration with immobilised biomass of algae or cyanobacteria, having a closed structure divided into segments and capsules of biomass of algae or cyanobacteria, **characterised in that** each of the capsules of biomass of algae or cyanobacteria (3) has a diameter between 5 mm and 40 mm and is individually supplied with light from a light source (6) by light tubes (5).

2. The photobioreactor according to claim 1, **characterised in that** the capsules of biomass of algae or cyanobacteria (3) in the photobioreactor segment lie freely on a perforated grid (2).

3. The photobioreactor according to claim 1, **characterised in that** light from the light source (6) is transferred via a separate single light tube (5) to the inside of each capsule of biomass of algae or cyanobacteria (3).

4. The photobioreactor according to claim 1, **characterised in that** the capsules of biomass of algae or cyanobacteria (3) are surrounded by a gaseous atmosphere.

5. The photobioreactor according to claim 1, **characterised in that** the capsules of biomass of algae or cyanobacteria (3) are wetted with a culture medium.

6. The photobioreactor according to claim 5, **characterised in that** the capsules of biomass of algae or cyanobacteria (3) are periodically wetted and flushed with the culture medium.

## Patentansprüche

1. Photobioreaktor für Biosequestration des CO₂ mit immobilisierter Algen-oder Cyanobakterienbiomasse, der eine geschlossene, in Segmente und Kapseln von Algen- oder Cyanobakterienbiomasse unterteilte Struktur aufweist, **dadurch gekennzeichnet, dass** jede der Kapseln von Algen- oder Cyanobakterienbiomasse (3) einen Durchmesser zwischen 5 mm und 40 mm hat und individuell mit Licht von einer Lichtquelle (6) durch Lichtleitungen (5) versorgt wird.

2. Photobioreaktor nach Anspruch 1, **dadurch gekennzeichnet, dass** die Kapseln von Algen- oder Cyanobakterienbiomasse (3) in dem Segment des Photobioreaktors auf einem Lochgitter (2) frei liegen.

3. Photobioreaktor nach Anspruch 1, **dadurch gekennzeichnet, dass** die Licht von der Lichtquelle (6) durch eine separate, einzelne Lichtleitung (5) in das Innere jeder Kapsel von Algen- oder Cyanobakterienbiomasse (3) zugeführt wird.

4. Photobioreaktor nach Anspruch 1, **dadurch gekennzeichnet, dass** die Kapseln von Algen- oder Cyanobakterienbiomasse (3) von einer Gasatmosphäre umgeben sind.

5. Photobioreaktor nach Anspruch 1, **dadurch gekennzeichnet, dass** die Kapseln von Algen- oder Cyanobakterienbiomasse (3) mit einem Nährmedium befeuchtet werden.

6. Photobioreaktor nach Anspruch 5, **dadurch gekennzeichnet, dass** die Kapseln von Algen- oder Cyanobakterienbiomasse (3) mit einem Nährmedium periodisch befeuchtet und gespült werden.

## Revendications

1. Un photobioréacteur pour la bioséquestration du CO₂ avec de la biomasse d'algues ou de cyanobactéries immobilisée, ayant une structure fermée divisée en segments et capsules de biomasse d'algues ou de cyanobactéries, **caractérisé en ce que** chacune des capsules de biomasse d'algues ou de cyanobactéries (3) a un diamètre compris entre 5 mm et 40 mm et est alimentée individuellement avec de la lumière provenant d'une source de lumière (6) par des tubes de lumière (5).

2. Le photobioréacteur selon la revendication 1, **caractérisé en ce que** les capsules de biomasse d'algues ou de cyanobactéries (3) dans le segment photobioréacteur reposent librement sur une grille perforée (2).

3. Le photobioréacteur selon la revendication 1, **caractérisé en ce que** la lumière provenant de la source de lumière (6) est transférée par l'intermédiaire d'un tube de lumière unique séparé (5) à l'intérieur de chaque capsule de biomasse d'algues ou de cyanobactéries (3).

4. Le photobioréacteur selon la revendication 1, **caractérisé en ce que** les capsules de biomasse d'algues ou de cyanobactéries (3) sont entourées par une atmosphère gazeuse.

5. Le photobioréacteur selon la revendication 1, **caractérisé en ce que** les capsules de biomasse d'algues ou de cyanobactéries (3) sont mouillées avec un milieu de culture.

6. Le photobioréacteur selon la revendication 5, **caractérisé en ce que** les capsules de biomasse d'algues ou de cyanobactéries (3) sont périodiquement mouillées et rincées avec le milieu de culture.
